# EUROPEAN PATENT APPLICATION

(11) **EP 1 820 534 A2**
(43) Date of publication of application: **22.08.2007**
(21) Application number: 07250491.3
(22) Date of filing: 07.02.2007
(51) Int. Cl.: A61N 1/375

(54) **Nano-titanium for making medical implantable hermetic feedthrough assemblies**

(30) Priority: 07.02.2006 US 765928 P
(71) Applicant: Greatbatch Ltd., Clarence, NY 14031 (US)
(72) Inventor: Fu, Richard, Ellicott City, MD 21042 (US); Lou, Huadong, College Park, MD 20740 (US); Frysz, Christine, Orchard Park, NY 14127 (US)
(74) Representative: Duckett, Anthony Joseph

(57) **Abstract**

Ferrules made of nano-titanium for incorporation into feedthrough filter capacitor assemblies are described. The feedthrough filter capacitor assemblies are particularly useful for incorporation into implantable medical devices such as cardiac pacemakers, cardioverter defibrillators, and the like, to decouple and shield internal electronic components of the medical device from undesirable electromagnetic interference (EMI) signals. Nano-titanium experiences significantly less grain growth after high temperature brazing in comparison to commercially pure (CP) titanium and the titanium alloy Ti-6Al-4V. For that reason, nano-titanium is an ideal material for use in implantable medical applications where high strength, structural integrity even after heating and corrosion resistance are desired.

## Description

### CROSS-REFERENCE TO RELATED APPLICATION

This application claims priority from provisional application Serial No. 60/765,928, filed February 7, 2006.

### BACKGROUND OF THE INVENTION

This invention relates generally to a hermetic feedthrough terminal pin assembly, preferably of the type incorporating a filter capacitor. More specifically, this invention relates to the manufacture of biocompatible metallic ferrules from nanotitanium. Preferably, the nano-titanium ferrules are incorporated into feedthrough filter capacitor assemblies, particularly of the type used in implantable medical devices such as cardiac pacemakers, cardioverter defibrillators, and the like, to decouple and shield internal electronic components of the medical device from undesirable electromagnetic interference (EMI) signals. The feedthrough assembly provides a hermetic seal that prevents passage or leakage of fluids into the medical device.

### SUMMARY OF THE INVENTION

A feedthrough filter capacitor assembly comprises an outer ferrule of titanium hermetically sealed to either an alumina insulator or fused glass dielectric material seated within the ferrule. The insulative material is also hermetically sealed to at least one terminal pin. A gold braze typically accomplishes these hermetic seals. That way, the feedthrough assembly prevents leakage of fluid, such as body fluid in a human implant application, past the hermetic seal at the insulator/ferrule and insulator/terminal pin interfaces. In a preferred form, a filter capacitor is mounted on the insulator and electrically connected to the terminal pins and to the ferrule to prevent unwanted EMI signals from traveling along the terminal pins and entering the interior of the medical device.

Titanium is used for the outer ferrule because it is chemically and biologically compatible with human fluids and tissue. However, during the high temperature gold braze process the grain structure of titanium can grow significantly. At room temperature, commercially pure (CP) titanium has a grain size of about 10 µm. After gold brazing, the grain size is typically from about 200 µm to about 600 µm. This magnitude of change can result in feedthrough geometry changes, secondary operations failure and degradation of ultimate and yield strength properties.

Therefore, there is a need for a new form of titanium that is useful in manufacturing ferrules for implantable medical devices, and the like. This need is predicated on the desirable biocompatibility of titanium along with its relatively light weight and corrosion resistance. The new form of titanium must have all of these attributes while maintaining its structural strength and dimensional integrity, even after high temperatures braze processing. The use of nano-titanium in the manufacture of implantable ferrules, and the like, fulfills these requirements.

These and other objects and advantages of the present invention will become increasingly more apparent by a reading of the following description in conjunction with the appended drawings.

### BRIEF DESCRIPTION OF THE DRAWINGS

FIG. 1 is a perspective view of a feedthrough assembly embodying the novel features of the invention.
FIG. 2 is an enlarged sectional view taken along line 2-2 of FIG. 1.
FIGs. 3A to 3C are microphotographs of the grain size of as received commercially pure titanium in comparison to the same material after having been subjected to various braze heating profiles.
FIGs. 4A to 4C are microphotographs of the grain size of as received nano-titanium in comparison to the same material after having been subjected to various braze heating profiles.
FIG. 5 is a cross-sectional view taken along line 5-5 of FIG. 2.
FIG. 6 is a cross-sectional view taken along line 6-6 of FIG. 2.

### DETAILED DESCRIPTION OF THE PREFERRED EMBODIMENTS

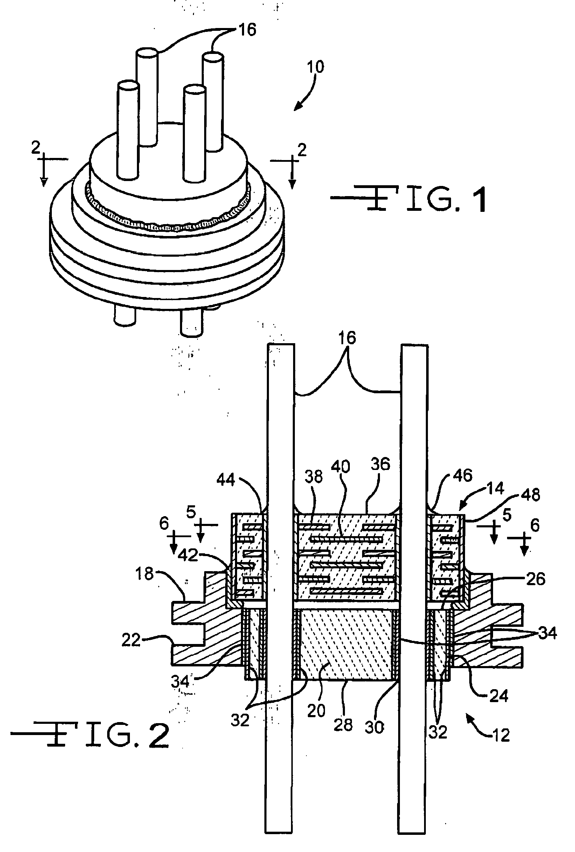

Referring now to the drawings, FIGs. 1 and 2 show an internally grounded feedthrough filter capacitor assembly 10 comprising a feedthrough 12 supporting a filter discoidal capacitor 14. The feedthrough filter capacitor assembly 10 is useful with medical devices, preferably implantable devices such as pacemakers, cardiac defibrillators, cardioverter defibrillators, cochlear implants, neurostimulators, internal drug pumps, deep brain stimulators, hearing assist devices, incontinence devices, obesity treatment devices, Parkinson's disease therapy devices, bone growth stimulators, and the like. The feedthrough 12 portion of the assembly 10 includes terminal pins 16 that provide for coupling, transmitting and receiving electrical signals to and from a patient's heart, while hermetically sealing the interior of the medical device against ingress of patient body fluids that could otherwise disrupt device operation or cause instrument malfunction. While not necessary for accomplishing these functions, it is desirable to attach the filter capacitor 14 to the feedthrough 12 for suppressing or decoupling undesirable EMI signals and noise transmission into the interior of the medical device along the terminal pins 16.

More particularly, the feedthrough 12 of the feedthrough filter capacitor assembly 10 comprises a ferrule 18 defining a bore surrounding an insulator 20. The ferrule 18 may be of any geometry, non-limiting examples being round, rectangle, and oblong. A surrounding flange 22 extends from the ferrule 18 to facilitate attachment of the feedthrough 10 to the casing (not shown) of, for example, one of the previously described implantable medical devices. The method of attachment may be by laser welding or other suitable methods.

The terminal pins 16 consist of niobium, tantalum, nickel-titanium (NITINOL^{®}), titanium, particularly beta titanium, titanium alloys, stainless steel, molybdenum, tungsten, platinum, platinum-iridium, palladium, palladium alloys, and combinations thereof.

The insulator 20 is of a ceramic material such as of alumina, zirconia, zirconia toughened alumina, aluminum nitride, boron nitride, silicon carbide, glass or combinations thereof. Preferably, the insulating material, is alumina, which is highly purified aluminum oxide, and comprises a sidewall 24 extending to a first upper side 26 and a second lower side 28. The insulator 20 is also provided with bores 30 that receive the terminal pins 16 passing there through. A layer of metal 32, referred to as metallization, is applied to the insulator sidewall 24 and the sidewall of the terminal pin bores 30 to aid a braze material 34 in hermetically sealing between the ferrule 18 and the insulator 24 and between the terminal pins 16 and the insulator 24, respectively.

Suitable metallization materials 32 include titanium, titanium nitride, titanium carbide iridium, iridium oxide, niobium, tantalum, tantalum oxide, ruthenium, ruthenium oxide, zirconium, gold, palladium, molybdenum, silver, platinum, copper, carbon, carbon nitride, and combinations thereof. The metallization layer may be applied by various means including, but not limited to, sputtering, electron-beam deposition, pulsed laser deposition, plating, electroless plating, chemical vapor deposition, vacuum evaporation, thick film application methods, and aerosol spray deposition, and thin cladding. Parylene, alumina, silicone, fluoropolymers, and mixtures thereof are also useful metallization materials.

Non-limiting examples of braze materials include gold, gold alloys, and silver. Then, if the feedthrough 10 is used where it will contact bodily fluids, the resulting brazes do not need to be covered with a biocompatible coating material. In other embodiments, if the brazes are not biocompatible, for example, if they contain copper, they are coated with a layer/coating of biocompatible/biostable material. Broadly, the biocompatibility requirement is met if contact of the braze/coating with body tissue and blood results in little or no immune response from the body, especially thrombogenicity (clotting) and encapsulation of the electrode with fibrotic tissue. The biostability requirement means that the braze/coating remains physically, electrically, and chemically constant and unchanged over the life of the patient.

Titanium is an electrically conductive material that is preferred for the ferrule 18. More particularly, commercially pure (CP) titanium is a desirable ferrule material because it is lightweight and chemically and biologically more compatible with human tissues than a commonly used titanium alloy designated Ti-6A1-4V. However, because commercially pure titanium experiences significant grain growth after high temperature brazing, its degraded mechanical properties and deformation behavior prevent it from being the ideal ferrule material.

Furthermore, titanium grain growth cannot be significantly reduced by changing the gold braze profile. A standard profile frequently used to gold braze a ferrule of commercially pure titanium to an alumina insulator is as follows: (1) 35°C/min to 1,055°C for 10 minutes, (2) 5°C/min to 1,065°C for 10 minutes, (3) 75°C/min to 1,090°C for 18 seconds, (4) power off and cool to 500°C, (5) liquid nitrogen purge cool to room temperature. An alternate method for brazing CP titanium to an alumina insulator is referred to as the "without soak braze profile" and is as follows: (1) 35°C/min to 1,055°C for 0.1 minutes, (2) 35°C/min to 1,065°C for 0.1 minutes, (3) 75°C/min to 1,090°C for 18 seconds, (4) power off and cool to 500°C, (5) liquid nitrogen purge cool to room temperature. This latter braze profile does not result in significantly reduced titanium grain growth.

On the other hand, although the titanium alloy Ti-6Al-4V is generally considered to be chemically inert, biocompatible with human tissue, and corrosion resistant to human body fluids and other corrosive environments, vanadium and aluminum potentially release alloy elements into the body.

Nano-titanium, defined as titanium having a grain size of less than about 1 µm, and preferably from about 0.10 µm to about 0.50 µm, is stronger than both commercially pure titanium and the titanium alloy Ti-6A1-4V, Table 1 compares typical mechanical properties of commercially pure titanium, titanium alloy Ti-6A1-4V and nano-titanium.

**Table 1**

| Mechanical Properties of Titanium | | | |
|---|---|---|---|
| | Ultimate | Yield Strength | Elongation |
| | | | (%) |
| | (ksi) | (ksi) | |
| CP Ti | 50-90 | 30-70 | 16-27 |
| Ti-6A1-4V | 125 | 115 | 12 |
| Nano-Ti | 125-160 | 115-152 | 6-12 |

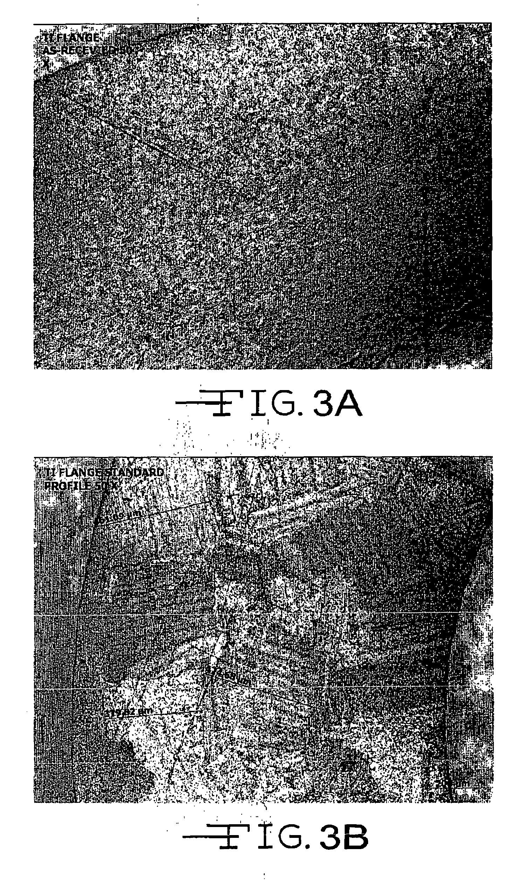

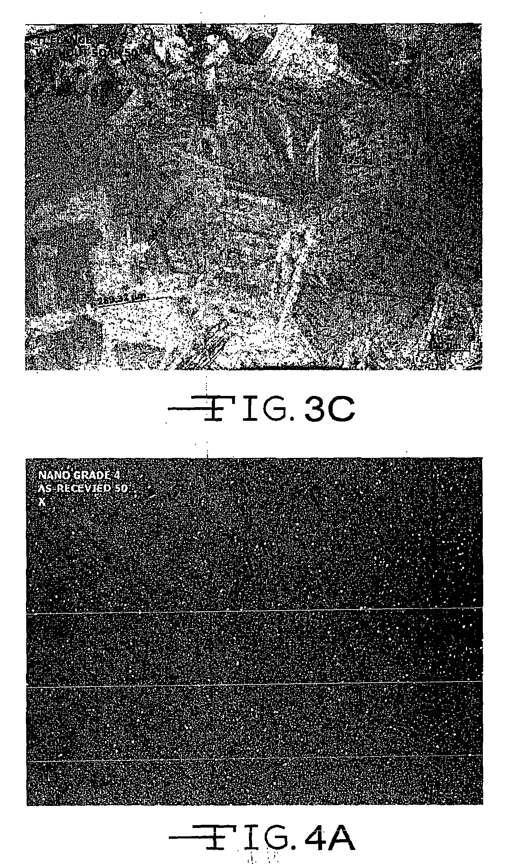

FIGs. 3A to 3C show CP grade 2 titanium microstructures before and after brazing. The grain size is about 10 µm before brazing and from about 200 µm to about 600 µm after brazing. In particular, FIG. 3A is a SEM photograph of as received CP titanium, FIG. 3B is a photograph of the same grade of titanium after having been subjected to the previously described standard braze profile and FIG. 3C is a photograph of CP grade 2 titanium after having been subjected to a "without soak braze profile." The significant growth in grain size is readily apparent regardless of the braze profile.

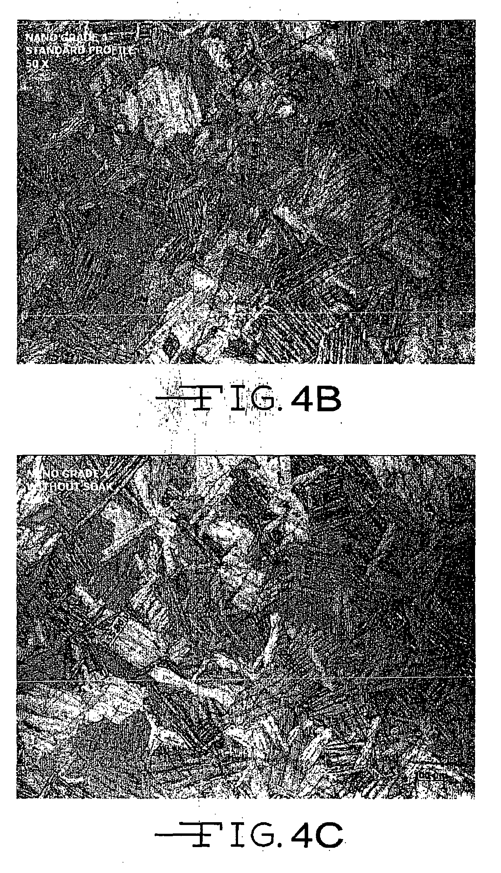

FIGs. 4A to 4C show nano-titanium microstructures before and after brazing. The grain size is about 0.26 µm before, and from about 80 µm to about 200 µm after brazing. In particular, FIG. 4A is a SEM photograph of as received nano-titanium, FIG. 4B is a photograph of the same grade of titanium after having been subjected to the previously described standard braze profile and FIG. 4C is a photograph of nano-titanium after having been subjected to a "without soak braze profile." While the grain size of the samples that were subjected to the respective braze profiles has increased from that of the as received sample, the magnitude of grain growth is significantly reduced from that of the CP grade 2 titanium shown in FIGs. 3B and 3C.

In that respect, the combination of biocompatibility, high strength, and lack of toxic alloying elements makes nanotitanium an attractive alternative to commercially pure titanium and the titanium alloy Ti-6Al-4V, particularly as a material for manufacturing ferrules for hermetically sealed feedthrough filter capacitor assemblies. Nano-titanium used for fabricating ferrules for medical implantable hermetic seal feedthroughs may be made by one of the following non-limiting processes: equal channel angular pressing (ECAP), cold rolling, cold extrusion, severe plastic deformation (SPD), and the like. The thusly processed nano-titanium body is then subjected to a machining process to provide the product ferrule.

Equal channel angular pressing (ECAP) is a processing procedure in which titanium is subjected to intense plastic straining but without the introduction of any change in the cross-sectional dimensions of the sample. This straining is achieved by simple shear by pressing the titanium through a die containing two channels, equal in cross-section, intersecting at an angle of Φ. An important characteristic of ECA pressing is that it provides the potential for refining the grain size of titanium down to a nanometer range of about 0.10 µm to about 0.5 µm. Homogeneous microstructures of ultrafine titanium grains may be achieved most readily when using a die with an internal angle of Φ = 90°.

A laser pyrolysis machine as described in U.S. Patent No. 6,193,936 to Gardner et al, entitled to "Reactant Delivery Apparatuses," which is incorporated herein by reference, can also be used to produce nano-titanium according to the present invention.

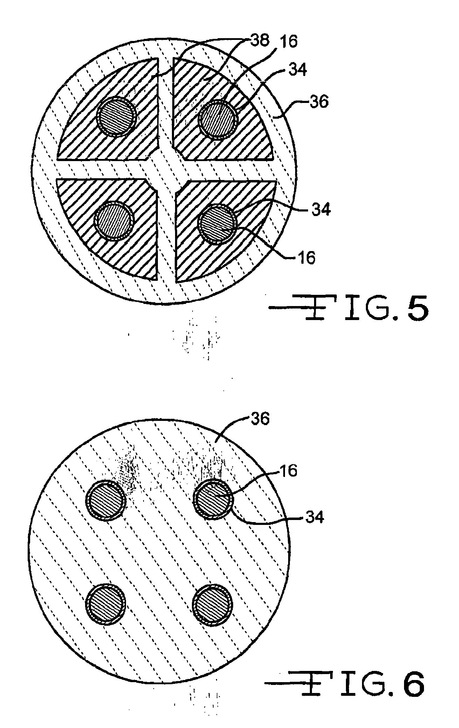

As further shown in FIGs. 1, 2, 5 and 6, a preferred form of the feedthrough filter capacitor assembly 10 includes a filter capacitor 14 that provides for filtering undesirable EMI signals before they can enter the device housing via the terminal pins 16. The filter capacitor 14 comprises a ceramic or ceramic-based dielectric monolith 36 having multiple capacitor-forming conductive electrode plates formed therein. The capacitor dielectric 36 preferably has a circular cross-section matching the cross-section of the ferrule 18 and supports a plurality of spaced-apart layers of first or "active" electrode plates 38 in spaced relationship with a plurality of spaced apart layers of second or "ground" electrode plates 40. The filter capacitor 14 is preferably joined to the feedthrough 12 adjacent to the insulator side 26 by an annular bead 42 of conductive material, such as a solder or braze ring, or a thermal-setting conductive adhesive, and the like. The dielectric 36 includes lead bores 44 provided with an inner surface metallization layer. The terminal pins 16 pass there through and are conductively coupled to the active plates 38 by a conductive braze material 46 contacting between the terminal pins 16 and the bore metallization. In a similar manner, the ground plates 40 are electrically connected through an outer surface metallization 48 and the conductive material 42 to the ferrule 18.

A method of making or forming a ferrule for a feedthrough by metal' injection molding is also described herein. In general, the metal injection molding process begins by designing and making a suitable mold. Next, titanium particles generally having spherical shapes with a nominal diameter of from about 0.10 µm to about 0.50 µm are mixed with a binder which may be a blend of polymers, wax and other materials. A thermal mechanical process is used to mix the combination of about 40% binder and about 60% titanium particles, by weight. The mixture is then pelletized and injected into a mold. This produces a "green part" which is typically about 19% to about 25% larger than the finished product. The green part is then subjected to a debinding process where about 90% of the polymer binding material is removed through thermal, solvent, or catalytic reactions. The resulting "brown part" is then sintered by heating it to about 96% of the melting point for titanium. Sintering shrinks the brown part by about 17% to about 22% to nearly full density. The ferrule is then complete with no further annealing steps being required.

In one illustrative method of the present invention, a ferrule 18 is produced through the process of metal injection molding, where the mold produces the surrounding flange 22 completely integral with main body of the ferrule and provides for any other design features as desired. In the preferred method, the pellatized titanium is mixed with a binder in a debinding system called catalytic debinding which yields ferrules that are particularly dimensionally stable. The debinding system is available from Phillips Origen Powder Metal Molding (Menomonie, Wis.). The molding is performed on a conventional molding press used for injection molding plastics, but with an altered profile on the screw. The mold is run hot to increase the flow rate of the material. Hot oil is used to heat the mold. The mold is preferably equipped with pressure transducers to indicate the pressure in the mold cavity and to thereby adjust molding parameters accordingly. The "green" ferrule undergoes debinding in a gas-tight oven at elevated temperature. The "brown" ferrule is then sintered in an atmosphere controlled high temperature oven.

While a preferred metal injection molding process has been described, it should be readily apparent that various metal injection molding processes may be used to produce a ferrule according to the present invention. The process described above is for illustrative purposes only.

It is appreciated that various modifications to the invention concepts described herein may be apparent to those of ordinary skill in the art without departing from the scope of the present invention as defined by the appended claims.

## Claims

1. A feedthrough assembly, which comprises:
a) an insulator of electrically non-conductive material having a height defined by an insulator sidewall extending to a first insulator end and a second insulator end, wherein the insulator has at least one terminal pin bore extending from the first end to the second end thereof;
b) a terminal pin received in the terminal pin bore, the terminal pin having a sidewall extending to opposed first and second ends disposed spaced from the respective first and second insulator ends;
c) a ferrule of an electrically conductive material and comprising a ferrule opening defined by a surrounding sidewall extending to a first ferrule end and a second ferrule end, wherein the insulator is supported in the ferrule opening;
d) a first braze material hermetically sealing the terminal pin to the insulator and a second braze material hermetically sealing the insulator to the ferrule; and
e) wherein the ferrule is of titanium having a grain size prior to brazing of less than about 1 µm.

2. The feedthrough assembly of claim 1 wherein the titanium has a grain size of from about 80 µm to about 200 µm.

3. The feedthrough assembly of claim 1 or claim 2 wherein the titanium has a grain size of about 0.10 µm to about 0.50 µm prior to being brazed to the insulator.

4. The feedthrough assembly of any of claims 1 to 3 wherein the titanium has an ultimate strength of from about 126 ksi to about 160 ksi.

5. The feedthrough assembly of any of claims 1 to 4 wherein the titanium has a yield strength of from about 126 ksi to about 160 ksi.

6. The feedthrough assembly of any of claims 1 to 5 wherein the titanium has an elongation of from about 6% to about 12%.

7. The feedthrough assembly of any of claims 1 to 6 including a filter capacitor electrically connected to the terminal pin and to the ferrule to decouple EMI signals. "

8. The feedthrough assembly of any of claims 1 to 7 wherein the terminal pin is selected from the group consisting of niobium, tantalum, nickel-titanium (NITINOL^{®}), titanium, beta titanium, titanium alloys, stainless steel, molybdenum, tungsten, platinum, platinum-iridium, palladium, palladium alloys, and combinations thereof.

9. The feedthrough assembly of any of claims 1 to 8 wherein the insulator is selected from the group consisting of alumina, zirconia, zirconia toughened alumina, aluminum nitride, boron nitride, silicon carbide, glass, and mixtures thereof.

10. The feedthrough assembly of any of claims 1 to 9 wherein the first and second braze materials are selected from the group consisting of gold, gold alloys, and silver.

11. The feedthrough assembly of any of claims 1 to 10 further including a metallization material covering the insulator sidewall and the terminal pin bore, the metallization material selected from the group consisting of titanium, titanium nitride, titanium carbide, iridium, iridium oxide, niobium, tantalum, tantalum oxide, ruthenium, ruthenium oxide, zirconium, gold, palladium, molybdenum, silver, platinum, copper, carbon, carbon nitride, and mixtures thereof.

12. A method for providing a feedthrough assembly, comprising the steps of:
a) providing titanium having a grain size of from about 0.10 µm to about 0.50 µm;
b) mixing the titanium with a binder;
c) pelletizing the titanium/binder mixture;
d) injecting the palletized titanium into a mold to thereby produce a first, "green" ferrule;
e) debinding the first ferrule to produce a second, "brown" ferrule; and
f) sintering the second ferrule to produce a product ferrule.

13. The method of claim 12 including providing the titanium by a process selected from the group consisting of equal channel angular pressing, cold rolling, cold extrusion, severe plastic deformation, and laser pyrolysis.

14. The method of claim 12 or claim 13 including selecting the binder from the group consisting of polymers and wax.

15. The method of any of claims 12 to 14 including providing the titanium/binder mixture comprising about 40% binder and 60% titanium, by weight.

16. The method of any of claims 12 to 15 including providing the first ferrule being from about 19% to about 25% larger than the product ferrule.

17. The method of any of claims 12 to 16 including removing about 90% of the binder from the first ferrule during the debinding step.

18. The method of any of claims 12 to 17 including reducing the size of the second ferrule by about 17% to about 22% during sintering to produce the product ferrule.

19. The method of any of claims 12 to 18 including incorporating the product ferrule into a feedthrough assembly according to claim 1.
